# FASCICULE DE BREVET EUROPEEN

(11) **EP 3 046 614 B1**
(45) Date de publication et mention de la délivrance du brevet: **09.05.2018**
(21) Numéro de dépôt: 14789885.2
(22) Date de dépôt: 12.09.2014
(51) Int. Cl.: A61M 27/00

(54) **VALVE DE DRAINAGE AJUSTABLE**
EINSTELLBARES DRAINAGEVENTIL
ADJUSTABLE DRAINAGE VALVE

(30) Priorité: 16.09.2013 FR 1358897
(43) Date de publication de la demande: 27.07.2016
(73) Titulaire: Sophysa, 91400 Orsay (FR)
(72) Inventeur: NEGRE, Philippe, F-75116 Paris (FR)
(74) Mandataire: Nony
(86) Numéro de dépôt international: PCT/IB2014/064476
(87) Numéro de publication internationale: WO 2015/036976

(56) Documents cités:
- EP-A1- 0 060 369
- EP-A1- 0 688 575
- EP-A1- 1 386 634
- EP-A2- 2 420 284
- US-A- 5 637 083
- US-A1- 2007 093 741

## Description

### DOMAINE TECHNIQUE

La présente invention a pour objet une valve de drainage destinée à des applications thérapeutiques, notamment le traitement de l'hydrocéphalie qui consiste à dériver du liquide céphalo-rachidien (LCR) contenu dans les ventricules de la cavité crânienne vers un site de résorption, par exemple la cavité péritonéale. La valve est contrôlable depuis l'extérieur pour modifier, à travers des tissus cutanés, le passage ou la distribution de ce liquide.

### ETAT DE LA TECHNIQUE

On connaît par le brevet européen EP 0 688 575 B1 une telle valve. Elle comporte un rotor sur lequel est fixé un ressort à lame courbe, le ressort à lame venant s'appliquer élastiquement contre une bille pour la maintenir contre un orifice d'entrée de la valve de manière à réguler le passage de liquide par cet orifice d'entrée. La rotation du rotor entraîne le glissement du point de contact de la bille sur le ressort à lame et donc une modification de la contrainte exercée par le ressort à lame sur la bille (correspondant à une pression d'ouverture). Le rotor peut être verrouillé/déverrouillé par attraction et/ou répulsion mutuelle de micro-aimants disposés sur le rotor.

On connaît également du brevet européen EP 1 604 703 ou des brevets américains US 7,422,566 B2 et US 8,322,365 une valve dont le rotor comporte un chemin de came à profil extérieur latéral ou radial, engendrant un mouvement de translation perpendiculaire à l'axe de rotation du rotor. Un organe de rappel élastique, fixé sur le corps de la valve et en appui sur la bille d'obturation de l'orifice d'entrée, est également en appui sur le chemin de came par un contact mobile. Dans cette configuration, l'organe de rappel élastique exerce une force de poussée comportant une composante centripète sur le chemin de came du rotor. La rotation du rotor permet ainsi une modification de la contrainte exercée par l'organe de rappel élastique sur la bille.

Par ailleurs, les demandes de brevet européen EP 2 008 683 A1 et EP 1 738 792 A1 décrivent une valve dont le rotor présente un chemin de came hélicoïdal ou axial, engendrant un mouvement de translation suivant le même axe que l'axe de rotation du rotor. Cette surface est sous forme de « marches d'escalier en colimaçon» sur lesquelles vient s'appuyer une première extrémité d'un organe de rappel élastique composé d'un ressort à lame monté en bascule entre ses deux extrémités, sur le corps de la valve. Comme précédemment, l'organe de rappel élastique exerce une force de poussée sur le chemin de came. La rotation du rotor permet de modifier la hauteur de la première extrémité du ressort, et ainsi la pression de l'autre extrémité du ressort sur une bille d'obturation. Un nombre limité de pressions d'ouverture est ainsi défini.

Les demandes EP 2 420 284, US 2007/093741, US 5 637 083, US 2005/0010159 A1, WO 02/47754 A1, WO 2006/091581 A1 et EP 1 512 428 A1 décrivent encore d'autres exemples de valves de drainage pour le traitement de l'hydrocéphalie.

Les valves doivent être adaptées en fonction des plages de pression d'ouverture envisagées : en particulier, dans certains cas d'hydrocéphalie, le neurochirurgien pourra avoir besoin de pressions spéciales, sortant de la plage standard. Dans d'autres cas, il souhaitera disposer d'un choix de pressions plus important dans une plage déterminée, c'est-à-dire séparées par un incrément plus petit.

Dès 1992, la société Sophysa a fabriqué des valves dites « à pressions spéciales », capables d'atteindre des pressions d'ouverture maximales élevées (SU8 200 : de 80 à 200 mm H₂O), voire très élevées (SU8 300 : de 50, 75, 95, 125, 150, 180, 220 et 300 mm H₂O, SU8 400 : de 75 à 380 mm H₂O). L'intérêt pour les neurochirurgiens de pouvoir disposer d'une pression d'ouverture maximale très élevée réside dans la possibilité d'arrêter temporairement le drainage du liquide céphalo-rachidien à travers la valve en s'approchant le plus possible d'une position de fermeture totale (position OFF) et de tester ainsi la «shunt indépendance » du patient, c'est-à-dire sa capacité à tolérer l'absence de valve.

En 1995, la société Sophysa a mis sur le marché un modèle Sophy® Mini SM8 couvrant une plage de pression standard élargie allant de 30 à 200 mm H₂O (30, 50, 70, 90, 110, 140, 170 et 200 mm H₂O), décliné à partir de 1996 en plusieurs modèles à pressions spéciales capables de répondre à la quasi-totalité des besoins cliniques exprimés par les neurochirurgiens : SM8-140 (10, 25, 40, 60, 80, 100, 120, 140 mm H₂O), SM8-300 (50, 75, 100, 125, 150, 180, 220, 300 mm H₂O) et SM8-400 (80, 120, 150, 190, 230, 270, 330, 400 mm H₂O). Le modèle SM8-400 a notamment permis au Dr. Takahashi (Department of Pédiatrie Neurosurgery, Hokkaido Children's Hospital and Médical Center, Japon) de mettre au point une technique de sevrage chez l'enfant consistant à augmenter par paliers successifs le niveau de pression d'ouverture jusqu'à atteindre 400 mm H₂O, à maintenir ce niveau très élevé pendant 6 à 24 mois et à procéder ensuite au retrait de la valve (Takahashi Y : « Withdrawal of shunt systems - Clinical use of the programmable shunt system and its effect on hydrocephalus in children ». Child's Nerv Syst (2001) 17 : 472-477). Il a par ailleurs été démontré que l'utilisation du modèle SM8-300 permettait de réduire les complications d'hyperdrainage pendant la période post-opératoire (Kordas M : « Expérience with SM8-300 Sophysa adjustable valve in adult chronic hydrocephalus ». Poster présenté au Hydrocephalus 2006, 6-9 Septembre, Göteborg, Suède). Le modèle SM8-140 permet quant à lui de traiter spécifiquement les patients atteints d'hydrocéphalie à basse pression (« Low pressure hydrocephalus »).

En raison d'une part de l'amplitude limitée des plages de pressions d'ouverture disponibles, et d'autre part de la limitation du nombre de pressions d'ouverture disponibles pour un modèle donné, notamment dans la plage des hautes pressions d'ouverture, aucune valve ne peut cependant aujourd'hui traiter tous les types d'hydrocéphalie. Le neurochirurgien doit donc sélectionner le modèle de valve en fonction de l'étiologie de chaque patient et de la stratégie de traitement envisagée. Ainsi, en cas d'erreur de diagnostic sur l'étiologie de l'hydrocéphalie ou d'évolution imprévisible de l'état du patient, peut-il être amené à explanter la valve pour la remplacer par un autre modèle, présentant une plage de pressions d'ouverture différente ou offrant un nombre de positions différent.

Un but de la présente invention est de proposer une valve ajustable sur une plage très étendue de pressions d'ouverture, afin de permettre aux neurochirurgiens de traiter tous les types d'hydrocéphalie avec un seul modèle de valve, et ce, quelle que soit la stratégie thérapeutique adoptée.

### RESUME DE L'INVENTION

L'invention a ainsi pour objet une valve de drainage destinée à être implantée sous la peau d'un patient et à drainer du liquide céphalo-rachidien, ladite valve comportant :
- un corps définissant une chambre dans laquelle débouchent un orifice d'entrée et un orifice de sortie du liquide céphalo-rachidien,
- un obturateur apte à obturer au moins partiellement, voire totalement, l'orifice d'entrée, de préférence une bille,
- un organe de rappel élastique agencé pour exercer une contrainte sur l'obturateur de manière à le pousser, de manière élastique, vers l'orifice d'entrée afin d'obturer ledit orifice d'entrée, la pression minimale permettant de déplacer l'obturateur pour dégager l'orifice d'entrée étant appelée « pression d'ouverture »,
- un rotor logé dans la chambre, apte à tourner autour d'un axe X entre deux positions extrêmes et comportant un chemin de came sur lequel prend appui l'organe de rappel élastique de sorte que la contrainte exercée par l'organe de rappel élastique sur l'obturateur est modifiée par la rotation du rotor.

Selon un premier aspect principal de l'invention, le chemin de came est défini par un profil intérieur, orienté vers l'axe X, de manière que l'organe de rappel élastique exerce une force, dite « force de traction », comportant une composante centrifuge par rapport à l'axe X, de préférence une force sensiblement centrifuge, sur le chemin de came.

Ladite force de traction évolue en sens inverse de la distance séparant l'axe X du point de contact de l'organe de rappel élastique avec le chemin de came. Ainsi l'effort de traction, et donc la pression d'ouverture résultant de la contrainte exercée par l'organe élastique sur l'obturateur, sont d'autant plus élevés que ce point de contact se rapproche de l'axe X et d'autant plus faibles qu'il s'en éloigne.

D'autre part, pour une même rotation angulaire du rotor, la longueur du chemin de came parcourue par le point de contact de l'organe de rappel élastique est d'autant plus importante que le point de contact est éloigné de l'axe X et d'autant plus faible qu'il s'en rapproche. Les positions angulaires successives correspondant à des points de contact éloignés de l'axe X sont donc séparées entre elles par un chemin de came plus long que celles correspondant à des points de contact proches de l'axe X. Elles peuvent donc être définies avec une plus grande précision mécanique, ce qui confère aux pressions d'ouverture correspondantes une meilleure précision. A l'inverse des configurations existantes avec came à profil extérieur qui procurent, pour un point de contact éloigné du centre du rotor, un maximum de précision dans les hautes pressions d'ouverture, la conception avec came à profil intérieur procure un maximum de précision dans les faibles pressions d'ouverture. Cette conception est donc mieux adaptée aux besoins physiologiques des patients en procurant une précision élevée dans les basses pressions, là où une différence de pression de 10 mm d'H₂O peut avoir un effet significatif sur l'état clinique du patient, comme dans certains cas d'hydrocéphalie à pression normale ou d'hydrocéphalie à basse pression, et une précision moindre dans les très hautes pressions, là où une variation de quelques dizaines de mm d'H₂O n'a qu'un impact limité sur l'état clinique du patient. Comme on le verra plus en détail dans la suite de la description, le chemin de came peut ainsi avantageusement être très étendu autour de l'axe X du rotor. Le réglage de la contrainte exercée par l'organe de rappel élastique sur l'obturateur, c'est-à-dire le réglage de la pression d'ouverture, peut donc être très précis et/ou la plage des pressions d'ouverture peut être très large.

Une valve selon l'invention peut encore notamment comporter une ou plusieurs des caractéristiques optionnelles suivantes, selon toutes les combinaisons possibles :
- Le chemin de came s'étend sur plus de 270°, sur plus de 300°, plus de 320°, plus de 340°, plus de 350°, plus de 355°, de préférence sur plus de 370°, plus de 540°, voire plus de 720°, ou plus de 1080° autour de l'axe X de rotation du rotor ; Dans un mode de réalisation, le chemin de came s'étend sur 360° ;
- Le chemin de came est adapté de manière à assurer des première et deuxième butées de l'organe de rappel élastique empêchant la poursuite de la rotation du rotor au-delà de première et deuxième positions extrêmes, dans les premier et deuxième sens de rotation du rotor, respectivement ;
- Le chemin de came ne présente pas de rupture de pente (ou point singulier), de préférence s'étend en spirale autour de l'axe X, la pente pouvant notamment être sensiblement constante ;
- L'organe de rappel élastique est monté en rotation, par rapport au corps, autour d'un axe Y sensiblement parallèle à l'axe X et comporte un bras de levier en contact mobile avec le chemin de came et un bras d'appui sur l'obturateur, les bras du levier et d'appui étant reliés entre eux par un pivot passant par l'axe Y, de préférence sensiblement parallèle à l'axe X ;
- Le bras de levier définit avec le bras d'appui un secteur d'angle α coupant l'axe X quelle que soit la position du rotor,
   la direction du bras de levier étant définie, lorsque la valve est observée suivant l'axe X, par la droite passant par l'axe Y et par le point d'appui du bras de levier sur le chemin de came, et
   la direction du bras d'appui étant définie, lorsque la valve est observée suivant l'axe X, par la droite passant par l'axe Y et le point d'appui du bras d'appui sur l'obturateur ;
- L'organe de rappel élastique comporte soit un bras de levier rigide et un bras d'appui flexible, de préférence sous la forme d'un ressort à lame, de préférence courbe, soit un bras de levier flexible, de préférence sous la forme d'une tige flexible, et un bras d'appui rigide, de préférence sous la forme d'une lame rigide, de préférence courbe ;
- Le bras de levier est en contact mobile avec le chemin de came et comporte une tige rigide ou flexible, de préférence mobile et/ou déformable dans un plan perpendiculaire à l'axe X, de préférence distinct et de préférence parallèle à un plan perpendiculaire de l'axe X et passant par le chemin de came, et un suiveur de came, de préférence sensiblement axial, en contact mobile avec le chemin de came ;
- L'organe de rappel élastique exerce ladite force de traction pour l'ensemble des positions angulaires dudit rotor ; Autrement dit, la force de traction comporte une composante s'écartant de l'axe X sur toute la longueur du chemin de came ;
- Le chemin de came est défini par une cavité ménagée dans le rotor, en particulier définie par une boutonnière, une rainure ou un évidement, ou par un bourrelet de matière ;
- L'organe de rappel élastique et le rotor sont conformés pour autoriser le réglage d'une pression d'ouverture de la valve sur une plage d'une amplitude de préférence supérieure à 150 mmH₂O, de préférence supérieure à 200 mmH₂O, supérieure à 300 mmH₂O, supérieure à 350 mmH₂, supérieure à 400 mmH₂O, voire supérieure à 500 mmH₂O, voire supérieure à 600 mmH₂O ;
- Le rotor peut être positionné, de préférence verrouillé, dans une pluralité de positions angulaires d'indexation prédéterminées ;
- Sur au moins une plage de pressions d'ouverture, de préférence sur l'ensemble des pressions d'ouverture, la différence de pression d'ouverture entre deux positions angulaires d'indexation successives, ou « incrément », est constant ou variable, l'incrément évoluant de préférence de manière sensiblement exponentielle au fur et à mesure de la rotation du rotor ;
- Le rotor peut être verrouillé dans vingt-quatre positions angulaires prédéterminées, séparées entre elles de 15°, une rotation dans le sens horaire du rotor, lorsque la face externe du rotor est observée, conduisant à une augmentation de la pression d'ouverture ;
- Le rotor comporte un dipôle magnétique formé de deux micro-aimants fixes ou mobiles linéairement par rapport au rotor suivant une direction sensiblement radiale par rapport à l'axe X et aptes à coopérer avec des moyens de verrouillage du rotor de manière à verrouiller ledit rotor dans une pluralité de positions angulaires d'indexation prédéterminées La position angulaire d'indexation, observée suivant l'axe X, est définie par l'angle β formé par l'intersection de la droite passant par l'axe X et le pôle Nord du dipôle magnétique, avec la droite passant par l'axe des deux orifices d'entrée et de sortie ;
- Les positions du rotor évoluent de manière continue et le gradient de la pression d'ouverture en fonction de la position angulaire du rotor est constant ou variable sur au moins une plage de pressions d'ouverture, de préférence sur l'ensemble des pressions d'ouverture, ledit gradient évoluant de préférence de manière sensiblement exponentielle au fur et à mesure de la rotation du rotor ;
- Le chemin de came est conformé pour définir plusieurs plages de pressions d'ouverture, la pression d'ouverture évoluant de manière différente en fonction de la plage de pressions d'ouverture considérée, l'incrément ou le gradient pouvant être notamment constants dans plusieurs plages de pressions d'ouverture, et en particulier dans des plages successives ;
- De préférence, l'incrément ou le gradient augmentent d'une plage à la suivante, de préférence de manière que la pression d'ouverture augmente de façon sensiblement exponentielle au fur et à mesure de la rotation du rotor ;
- L'axe de rotation du rotor est excentré par rapport au centre de la chambre dans laquelle le rotor est logé et/ou par rapport à l'axe reliant les orifices d'entrée et de sortie du liquide céphalo-rachidien.

Les caractéristiques décrites ci-dessus peuvent être combinées entre elles ou avec une ou plusieurs des caractéristiques ci-dessous.

Suivant un deuxième aspect principal de l'invention, le chemin de came du rotor s'étend sur 360°, de préférence sur plus de 360°, plus de 370° ou plus de 400°, plus de 720°, voire plus de 1080° autour de l'axe du rotor.

Selon un troisième aspect principal de l'invention, l'organe de rappel élastique comporte un suiveur de came qui est guidé linéairement sur le chemin de came. Dans une position angulaire du rotor déterminée, le suiveur de came de l'organe de rappel élastique ne peut donc être écarté radialement du chemin de came. Un tel guidage peut être en particulier obtenu avec une boutonnière.

Selon un quatrième aspect principal de l'invention, le chemin de came est conformé de manière que la pression d'ouverture ne varie pas proportionnellement avec la position angulaire du rotor. De préférence, la pression d'ouverture évolue plus rapidement que la position angulaire du rotor. Autrement dit, à partir d'une position angulaire correspondant à une pression d'ouverture déterminée, un déplacement angulaire du rotor déterminé, par exemple de 15°, produit une variation de la pression d'ouverture d'autant plus élevée que la pression d'ouverture initiale est grande, de préférence une variation exponentielle.

Sauf incompatibilité technique, une caractéristique d'une valve selon un aspect principal de l'invention peut être appliquée à une valve selon un autre aspect principal de l'invention.

### DEFINITIONS

Par définition, une pression d'ouverture correspond, dans une position du rotor, à la pression minimale nécessaire pour déplacer l'obturateur à l'encontre de la contrainte exercée par l'organe de rappel élastique sur l'obturateur.

Par « comportant un » ou « comprenant un », il y a lieu de comprendre « comportant au moins un », sauf indication contraire.

### BREVE DESCRIPTION DES FIGURES

L'invention pourra être mieux comprise à la lecture de la description détaillée qui va suivre, non limitative de celle-ci, et à l'examen du dessin annexé, dans lequel :
- la figure 1 représente, schématiquement, une valve de drainage conforme à l'invention, dans une position correspondant à une pression d'ouverture minimale, observée selon l'axe X par l'observateur Oₐ, à travers la face interne du corps 2,
- la figure 2 représente, schématiquement, la valve de la figure 1 suivant la coupe A-A dans le plan P₁ représenté sur la figure 1,
- la figure 3 représente, schématiquement, la valve des figures 1 et 2, observée selon l'axe X, à travers la face externe du corps 2, par l'observateur O_{b}, à l'exclusion du cadran gradué radio-opaque 122 et de l'indicateur radio-opaque 124 du rotor,

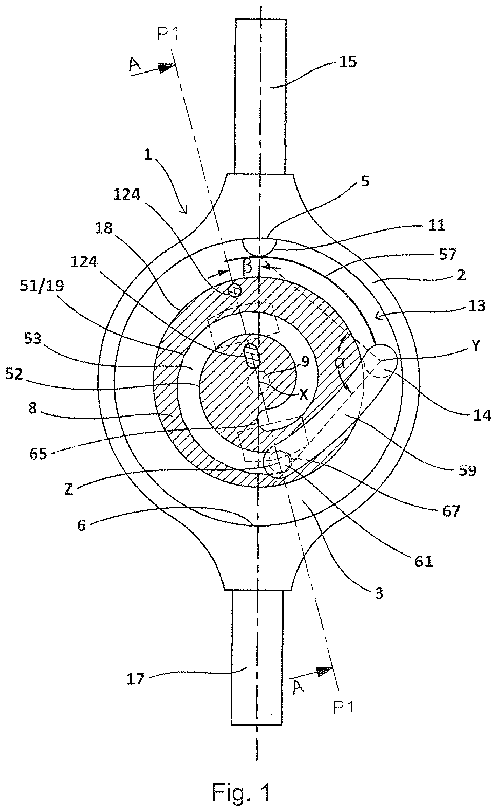

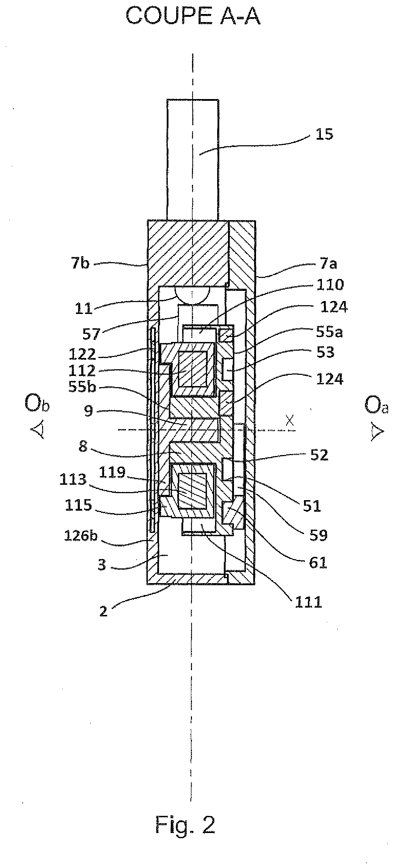

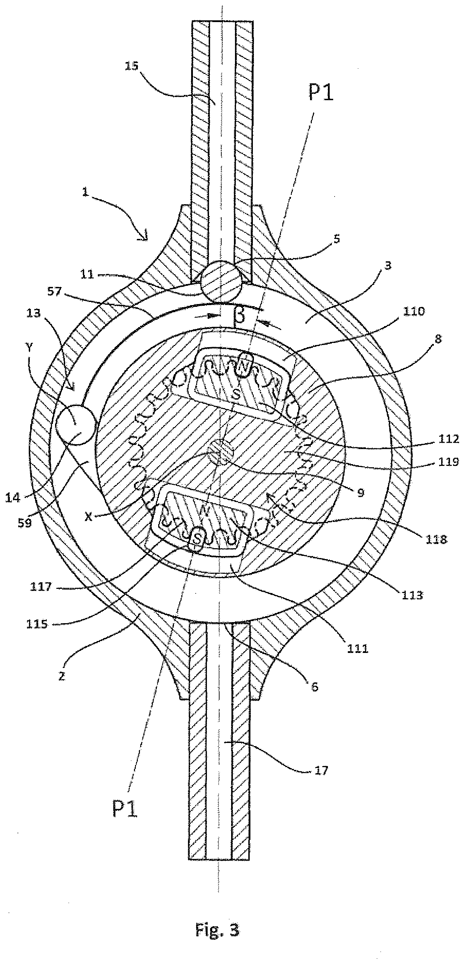

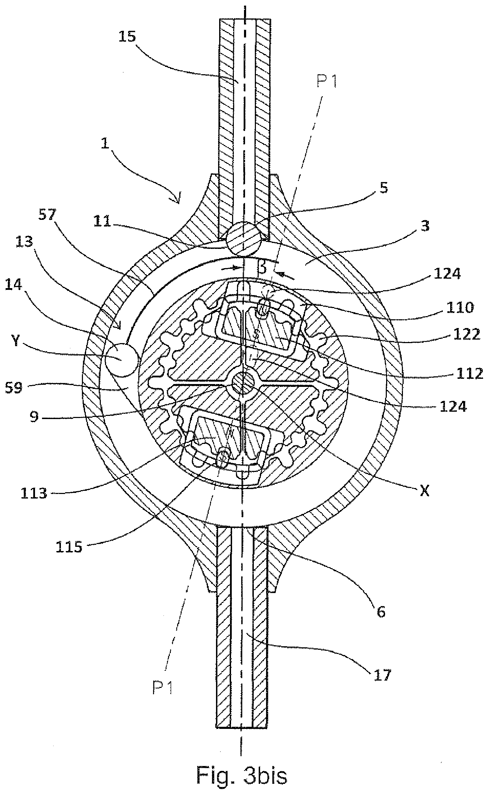

- la figure 3 bis représente, schématiquement, la valve des figures 1 et 2, observée selon l'axe X, à travers la face externe du corps 2, par l'observateur O_{b}, à l'exclusion des encoches 117 de la partie centrale 119,

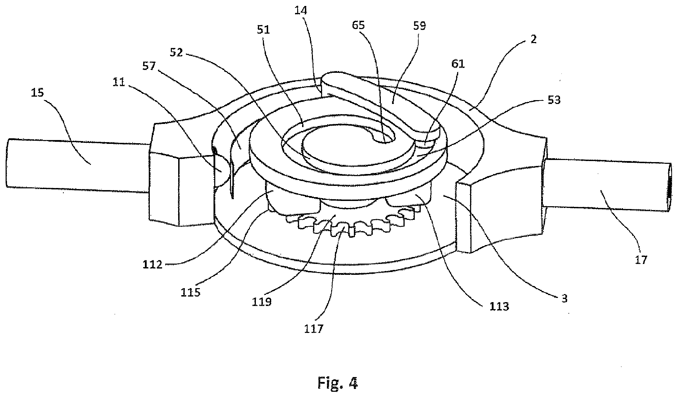

- la figure 4 représente, schématiquement en perspective et en écorché, la valve des figures 1, 2 et 3, à l'exclusion du cadran gradué radio-opaque 122 et de l'indicateur radio-opaque 124 du rotor,

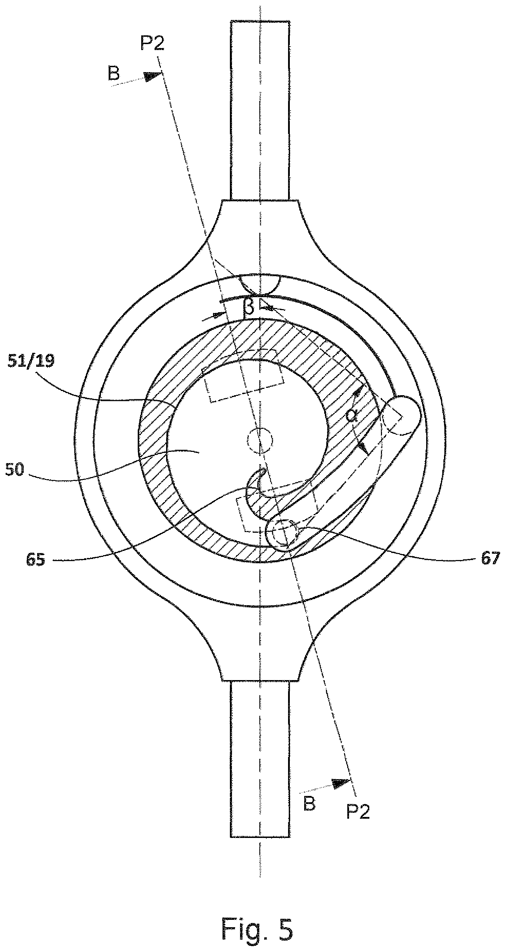

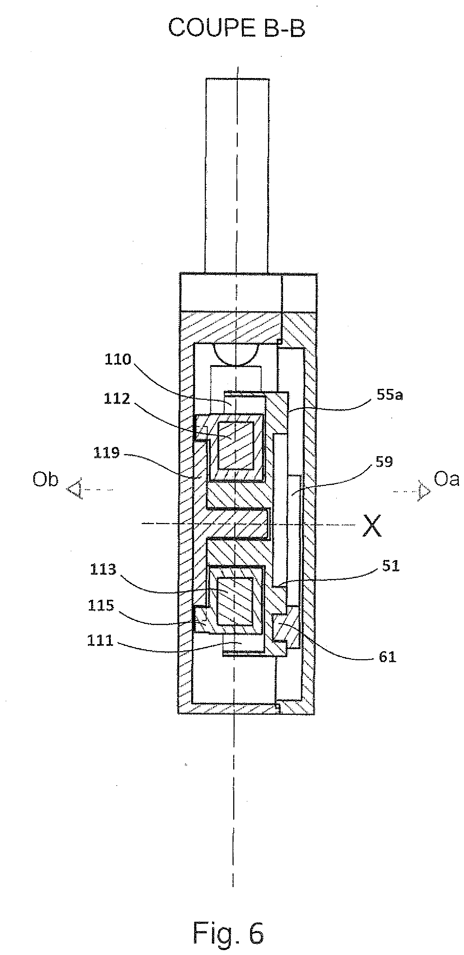

- la figure 5 représente, schématiquement, observé selon l'axe X par l'observateur Oₐ (voir figure 6), un autre mode de réalisation d'une valve selon l'invention, dans une position correspondant à une pression d'ouverture minimale,
- la figure 6 représente, schématiquement, suivant la coupe B-B dans le plan P₂ (voir figure 5), la valve représentée sur la figure 5,

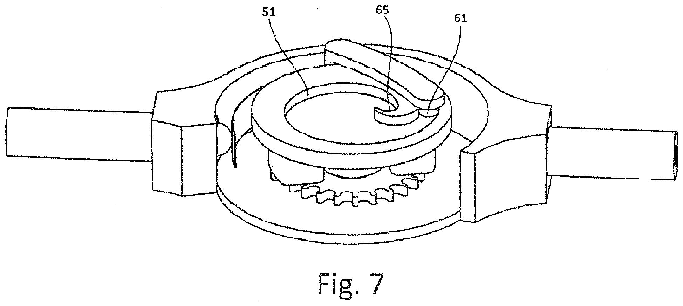

- la figure 7 représente, schématiquement en perspective et en écorché, la valve représentée sur les figures 5 et 6,

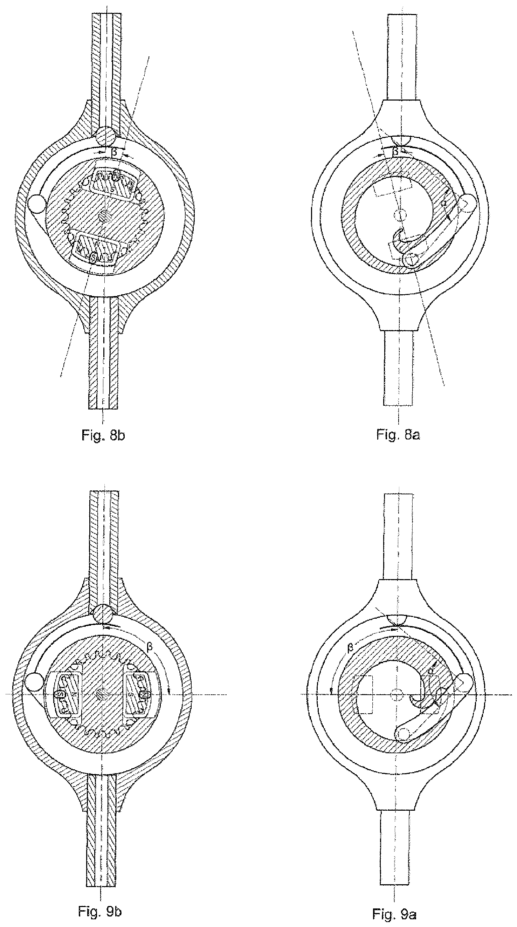

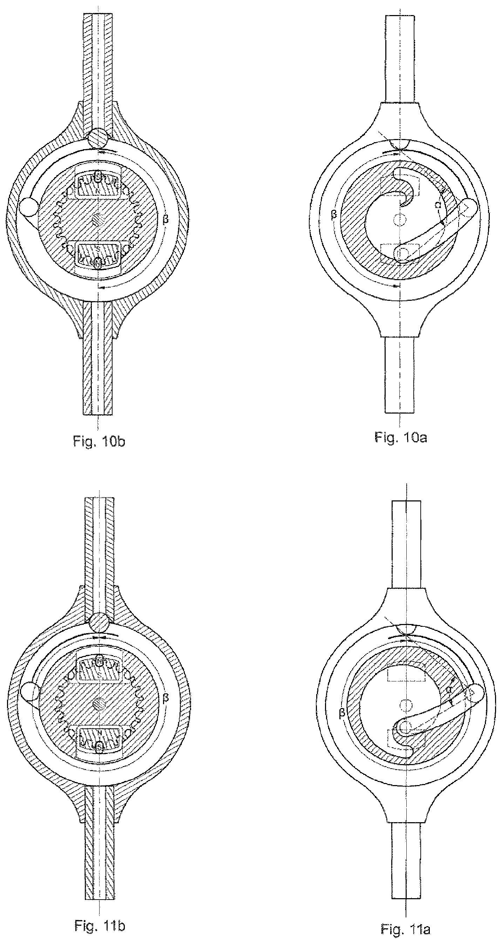

- les figures 8, 9, 10 et 11 représentent la valve de la figure 7, dans des positions angulaires du rotor correspondant, sur un cadran de 24 positions, aux positions 1, 6, 12 et 24, (les indices a et b correspondant à des observations, par les observateurs Oₐ et O_{b}, respectivement, c'est-à-dire par les faces interne et externe du corps, respectivement.

Sur les différentes figures, des références identiques ont été utilisées pour désigner des organes identiques ou analogues.

### DESCRIPTION DETAILLEE

On a représenté sur les figures des valves de drainage 1 conformes à l'invention, comportant chacune un corps 2 définissant une chambre 3 dans laquelle des orifices d'entrée 5 et de sortie 6 débouchent. La chambre 3 est de préférence sensiblement symétrique, en particulier par rapport à l'axe reliant les orifices d'entrée 5 et de sortie 6. De préférence, elle est sensiblement cylindrique d'axe X.

Le corps 2 comporte des faces interne 7a et externe 7b destinées, après implantation sous la peau d'un patient, à être orientées vers l'intérieur et l'extérieur du corps du patient, respectivement.

Un rotor 8, monté à rotation autour d'un pivot 9 d'axe X du corps, un obturateur constitué d'une bille 11 et un organe de rappel élastique 13 appuyant élastiquement sur la bille 11 pour la maintenir contre l'orifice d'entrée 5, sont logés dans la chambre 3.

Un conduit d'entrée 15 et un conduit de sortie 17 sont fixés sur le corps 2 et débouchent respectivement par les orifices d'entrée 5 et de sortie 6. Le conduit d'entrée 15 et le conduit de sortie 17 peuvent être connectés respectivement à un cathéter d'amenée et un cathéter d'évacuation de liquide, non représentés.

Le rotor 8 comporte une surface latérale extérieure 18, définissant son épaisseur, s'étendant sensiblement parallèlement à l'axe X.

La surface latérale extérieure du rotor peut être de forme quelconque.

Dans un mode de réalisation, le contour de la surface latérale extérieure du rotor 8 est sensiblement circulaire (observé selon l'axe X de rotation du rotor), de sorte que la distance entre cette surface et le point d'appui de l'organe de rappel élastique sur l'obturateur est sensiblement constante quelle que soit la position angulaire du rotor. L'encombrement radial de la valve en est réduit.

De préférence, le rotor 8 est fixe par rapport au corps suivant l'axe X de rotation du rotor.

De préférence, le corps n'est sensiblement pas déformable par pression manuelle (sans outil, à la seule force des mains).

Le chemin de came 19 est défini par l'ensemble des points de contact entre le rotor et l'organe de rappel élastique lors d'une rotation la plus grande possible du rotor.

La distance entre le point d'appui de l'organe de rappel élastique sur le chemin de came et l'axe de rotation du rotor varie en fonction de la position angulaire dudit rotor, ce qui permet de modifier la force exercée par l'organe de rappel élastique sur la bille 11.

Selon l'invention, le chemin de came est conformé de sorte que l'organe de rappel élastique exerce une force comportant une composante centrifuge, de préférence une force sensiblement centrifuge, sur le chemin de came. Le chemin de came est ainsi défini par un profil intérieur du rotor, orienté vers l'axe X.

De préférence, l'organe de rappel élastique exerce sur le chemin de came une force de traction quelle que soit la position angulaire du rotor. Dans un mode de réalisation, il existe cependant une position d'ouverture totale dans laquelle l'organe de rappel élastique n'exerce pas de force de traction sur le chemin de came, et donc n'exerce aucune contrainte sur l'obturateur.

Le chemin de came peut être en particulier une surface latérale ou radiale, sensiblement parallèle à l'axe de rotation du rotor.

De préférence, le chemin de came est conformé pour que le contact avec l'organe de rappel élastique soit permanent et continu (sans « saut ») lors de la rotation du rotor. L'encombrement de la valve en est amélioré.

La forme du chemin de came n'est pas limitative.

Le chemin de came peut notamment être défini par un trou dans le rotor, traversant ou non, en particulier une boutonnière (Fig. 1), une rainure ou un évidement (Fig. 5), ou par un bourrelet de matière.

Le chemin de came peut s'étendre jusqu'à 360° autour de l'axe du rotor. De préférence, le chemin de came s'étend sur plus de 360° autour de l'axe du rotor.

La pente du chemin de came, qui correspond à la vitesse d'évolution de la pression d'ouverture par degré de rotation du rotor, peut être constante ou augmenter à mesure que le rotor tourne. La forme du chemin de came est adaptée à la vitesse d'évolution de la pression d'ouverture souhaitée.

Dans un mode de réalisation, la pente du chemin de came diminue, optionnellement par paliers, à mesure que le rotor approche de sa position angulaire extrême correspondant à une pression d'ouverture minimale.

La valve selon l'invention peut être pourvue ou dépourvue d'un moteur (non représenté) pour entraîner en rotation le rotor. En particulier, le rotor peut être entraîné par un dipôle magnétique (micro-aimants fixes ou mobiles) ou par un moteur pas à pas, ou encore par un moteur piézo-électrique, ou plus généralement par tout type de micro-moteur implantable. En l'absence de moteur, la rotation du rotor peut être effectuée manuellement, de préférence par couplage magnétique du rotor avec un aimant manipulé par l'utilisateur.

La rotation du rotor 8 permet de modifier la pression d'ouverture.

L'organe de rappel élastique 13 est de préférence monté à rotation par rapport au corps 2 de valve, autour d'un axe Y, de préférence parallèle à l'axe X, de préférence à la manière d'une bascule, une première extrémité de l'organe de rappel élastique prenant appui sur l'obturateur et une deuxième extrémité de l'organe de rappel élastique prenant appui sur le chemin de came.

Il peut comporter un bras d'appui 57 rigide ou, de préférence élastique, en appui sur l'obturateur, de préférence courbe de manière à longer sensiblement la surface latérale extérieure 18 du rotor, et un bras de levier 59 élastique ou, de préférence, rigide, en appui sur le chemin de came, l'appui du bras de levier sur le chemin de came agissant sur la contrainte exercée par le bras d'appui sur l'obturateur. Au moins un desdits bras d'appui et bras de levier est rigide, l'autre étant souple.

La rigidité et l'élasticité du bras d'appui et du bras de levier s'évaluent au regard de leur capacité à être fléchis dans un plan perpendiculaire à l'axe X de rotation du rotor. La rigidité peut être par exemple obtenue au moyen d'une nervure ou d'une plaque s'étendant sensiblement perpendiculairement à l'axe X alors que la souplesse peut être par exemple obtenue en utilisant une lame s'étendant sensiblement parallèlement à l'axe X de rotation du rotor.

Le bras d'appui peut comporter ou être constitué par un ressort à lame, de préférence courbe. Un ressort à lame courbe confère avantageusement une grande compacité. Sous l'effet de la tension résultant de l'augmentation de la pression d'ouverture, le ressort à lame peut se déformer radialement vers l'extérieur, dans un plan perpendiculaire à l'axe X.

De préférence, la paroi latérale de la chambre dans laquelle est logé le rotor présente un renfoncement permettant d'éviter que, lors de sa déformation, le ressort à lame courbe n'entre en contact avec elle. Dans un mode de réalisation, l'axe de rotation du rotor est décalé par rapport au centre de la chambre 3 et/ou par rapport à l'axe reliant les orifices d'entrée 5 et de sortie 6.

L'excentration ou ledit décalage est de préférence supérieur à 0,3 mm, de préférence supérieur à 0,5 mm, de préférence supérieur à 0,8 mm, de préférence encore supérieur ou égal à 1 mm La compacité est alors optimale.

Le bras de levier peut être composé d'une tige s'étendant de préférence dans un plan perpendiculaire à l'axe X, apte à pivoter autour de l'axe Y, et d'un suiveur de came, de préférence sensiblement axial, en contact mobile avec le chemin de came.

Le suiveur de came est de préférence formé d'un pion 61, de préférence cylindrique, fixé sur ladite tige, ou d'un galet de préférence monté à rotation autour d'un axe Z parallèle à l'axe X. Le pion 61 s'étend de préférence sensiblement parallèlement à l'axe Y, de préférence perpendiculairement au bras de levier 59, de manière à ce que l'organe de rappel élastique 13 chevauche le rotor 8, de préférence dans toutes les positions du rotor 8. Autrement dit, l'organe de rappel élastique 13 présente une forme qui lui permet de crocheter le rotor 8 de manière à venir prendre appui sur le profil intérieur (orienté vers l'axe X).

Le bras de levier 59 s'étend, dans le prolongement du bras d'appui 57, en formant de préférence avec le bras d'appui 57 un angle α de préférence supérieur à 70°, supérieur à 80°, pour la position angulaire du rotor correspondant à la pression d'ouverture minimale. L'axe Y, défini par un pivot 14, passe par la jonction entre le bras d'appui 57 et le bras de levier 59.

Cette configuration « ouverte », avec les deux bras de l'organe de rappel élastique disposés de part et d'autre de l'axe X de rotation du rotor, permet avantageusement d'utiliser un chemin de came de grande longueur et un organe de rappel élastique, et en particulier un bras d'appui, de grande longueur. Il est ainsi possible, avec un encombrement réduit, de régler la pression d'ouverture de la valve sur une très large plage et/ou avec une très bonne précision.

Dans le mode de réalisation préféré, le bras d'appui 57 est un ressort à lame courbe et le bras de levier est rigide. Dans un autre mode de réalisation, le bras d'appui est une lame rigide le bras de levier est une tige flexible. L'axe Y est de préférence disposé au plus près, par exemple à moins de 2mm, ou moins de 1 mm, de la périphérie du rotor, de préférence de manière à ce que le bras d'appui 57 s'étende, de préférence en regard de la surface latérale extérieure du rotor, sur un secteur angulaire de préférence de plus de 70°. Cette configuration permet de réduire l'encombrement radial du corps de valve, tout en optimisant la longueur des deux bras de l'organe de rappel élastique. Cette optimisation permet d'étendre considérablement la plage de réglage des pressions d'ouverture, de couvrir, avec une même valve, la quasi-totalité des plages de pressions d'ouverture actuellement souhaitées, soit une amplitude de préférence égale ou supérieure à 400 mm H₂O, et d'autoriser des incréments fixes ou variables entre chaque position.

Comme représenté sur les figures 3 et 4 en particulier, le rotor comporte de préférence au moins un, de préférence au moins deux micro-aimants 112 et 113, de préférence deux micro-aimants mobiles linéairement par rapport au rotor suivant une direction sensiblement radiale par rapport à l'axe X et aptes à coopérer avec des moyens de verrouillage du rotor dans une position angulaire prédéterminée.

Le rotor peut comporter un ou plusieurs, en particulier deux logements 110 et 111 aptes à recevoir chacun un micro-aimant 112 et 113 respectivement. Chaque micro-aimant peut être agencé de manière à pouvoir coulisser linéairement dans le logement correspondant suivant une direction sensiblement radiale.

Chaque micro-aimant peut comporter un relief de verrouillage 115. Ce relief peut comporter par exemple un ergot cylindrique.

Chaque relief peut être apte à s'engager dans une encoche 117 de moyens de verrouillage 118.

Les moyens de verrouillage peuvent comporter une partie centrale 119 fixe par rapport au corps et sur la périphérie de laquelle sont réalisées les encoches. Ces encoches peuvent être régulièrement réparties tout autour de l'axe du rotor.

Grâce à un dispositif de réglage externe, il peut être possible de déplacer les micro-aimants simultanément dans leurs logements respectifs, radialement vers l'extérieur, afin de désengager les reliefs des encoches. Ce désengagement permet de faire tourner le rotor autour de l'axe de rotation d'une position angulaire d'indexation vers une autre. Le dispositif de réglage externe permet également de repositionner les micro-aimants dans une position verrouillée dans laquelle les reliefs sont engagés dans les encoches.

Il est possible de se référer au brevet EP 1 604 703 B1, pour plus de détails quant au fonctionnement du blocage magnétique à l'aide des deux micro-aimants et au brevet EP 0 688 575 B1, pour plus de détails quant à la structure du dispositif de réglage externe.

De préférence, le rotor ne peut adopter qu'un nombre limité de positions angulaires, dites « positions angulaires d'indexation ». De préférence, l'écart angulaire entre deux positions angulaires d'indexation du rotor est constant.

La différence de pression d'ouverture correspondant à deux positions angulaires d'indexation successives quelconque, ou « incrément », est de préférence inférieure à 50 mmH₂O, 40 mmH₂O, 30 mmH₂O, 20 mmH₂O, 10 mmH₂O, voire 5 mmH₂O.

En fonction de la forme du chemin de came, il est possible d'avoir différentes évolutions d'incréments et donc d'obtenir différentes courbes de variation de pression d'ouverture en fonction des positions angulaires du rotor.

Dans une première configuration, les pressions d'ouverture correspondant à chaque position angulaire d'indexation sont séparées par un incrément fixe, sensiblement égal à la plage de pressions d'ouverture divisée par le nombre d'intervalles entre chaque position. Par exemple, pour une valve couvrant une plage de pressions d'ouverture de 460 mm H₂O, entre 20 mm H₂O et 480 mm H₂O, avec 24 positions d'indexation, donc 23 intervalles, l'incrément sera de 460/23 = 20 mm H₂O. La pression d'ouverture de la valve varie donc linéairement en fonction de la position angulaire d'indexation, alors que le pourcentage de variation de pression d'ouverture d'une position à l'autre, très élevé dans la plage des basses pressions, devient particulièrement faible dans la plage des hautes pressions d'ouverture. Ainsi, le simple passage de la position 1 (20 mm) à la position 2 (40 mm) représente un doublement de la résistance de la valve (+100%), susceptible d'impacter défavorablement l'état clinique de certains patients. Inversement, le passage de la position 23 (460 mm) à la position 24 (480 mm) ne représente qu'une augmentation de 4%, dont l'impact sur l'état clinique du patient risque d'être relativement limité.

Le tableau 1 illustre cette configuration :

**Tableau 1**

| Position | Pression | Incrément fixe | |
|---|---|---|---|
| | mm H₂O | mm H₂O | % variation |
| 1 | 20 | | |
| 2 | 40 | 20 | 100% |
| 3 | 60 | 20 | 50% |
| 4 | 80 | 20 | 33% |
| 5 | 100 | 20 | 25% |
| 6 | 120 | 20 | 20% |
| 7 | 140 | 20 | 17% |
| 8 | 160 | 20 | 14% |
| 9 | 180 | 20 | 13% |
| 10 | 200 | 20 | 11% |
| 11 | 220 | 20 | 10% |
| 12 | 240 | 20 | 9% |
| 13 | 260 | 20 | 8% |
| 14 | 280 | 20 | 8% |
| 15 | 300 | 20 | 7% |
| 16 | 320 | 20 | 7% |
| 17 | 340 | 20 | 6% |
| 18 | 360 | 20 | 6% |
| 19 | 380 | 20 | 6% |
| 20 | 400 | 20 | 5% |
| 21 | 420 | 20 | 5% |
| 22 | 440 | 20 | 5% |
| 23 | 460 | 20 | 5% |
| 24 | 480 | 20 | 4% |

Dans une deuxième configuration, la rotation du rotor d'une position angulaire d'indexation à la suivante entraine, de préférence, à chaque changement de position angulaire d'indexation tendant à augmenter la pression d'ouverture, une augmentation de la pression d'ouverture d'autant plus élevée que la position angulaire d'indexation initiale correspond à une pression d'ouverture élevée.

Afin de mieux répondre aux besoins physiologiques des patients, il est en effet préférable d'avoir un incrément dont la valeur est fonction du niveau de pression d'ouverture ou de la plage de pressions d'ouverture concernée, de manière à minimiser le pourcentage de variation dans les plages de basses pressions d'ouverture et à le maximiser dans les plages de hautes pressions d'ouverture. Avantageusement, l'utilisateur peut ainsi régler avec une meilleure précision la pression d'ouverture quand la pression d'ouverture est peu élevée (« basse pression »), c'est-à-dire précisément dans la plage de pressions d'ouverture pour laquelle une variation de pression d'ouverture a le plus d'impact sur le patient. Une telle valve est donc beaucoup mieux adaptée que les valves de la technique antérieure à la physiologie du patient.

Les deux évolutions d'incréments suivantes sont préférées :
Dans une première configuration d'incrément variable préférée, l'incrément varie progressivement sur toute la plage de pressions, de préférence augmente avec la pression d'ouverture de manière à ce que la pression d'ouverture varie de façon sensiblement exponentielle en fonction de la position angulaire d'indexation. Par exemple, l'incrément peut varier progressivement de 5 à 41 mm sur une plage de 400 mm H₂O allant de 20 mm à 420 mm H₂O. L'augmentation de la pression d'ouverture entre la position 1 (20 mm) et la position 2 (25 mm) n'est ici que de 25%, alors que l'augmentation de la pression d'ouverture entre la position 23 (379 mm) et la position 24 (420 mm) est portée à 10,8%. Une valve selon l'invention permet ainsi une grande précision dans les basses pressions d'ouverture.

Le tableau 2 illustre cette configuration :

**Tableau 2**

| Position | Pression | Incrément progressif | |
|---|---|---|---|
| | mm H₂O | mm H₂O | % variation |
| 1 | 20 | | |
| 2 | 25 | 5,0 | 25,0% |
| 3 | 31 | 5,5 | 22,0% |
| 4 | 37 | 6,1 | 19,8% |
| 5 | 43 | 6,7 | 18,2% |
| 6 | 51 | 7,3 | 17,0% |
| 7 | 59 | 8,1 | 16,0% |
| 8 | 67 | 8,9 | 15,1% |
| 9 | 77 | 9,8 | 14,5% |
| 10 | 88 | 10,7 | 13,9% |
| 11 | 100 | 11,8 | 13,4% |
| 12 | 113 | 13,0 | 13,0% |
| 13 | 127 | 14,3 | 12,7% |
| 14 | 143 | 15,8 | 12,4% |
| 15 | 160 | 17,3 | 12,1% |
| 16 | 179 | 19,1 | 11,9% |
| 17 | 200 | 21,0 | 11,7% |
| 18 | 223 | 23,1 | 11,5% |
| 19 | 249 | 25,4 | 11,4% |
| 20 | 277 | 28,0 | 11,2% |
| 21 | 308 | 30,8 | 11,1% |
| 22 | 341 | 33,9 | 11,0% |
| 23 | 379 | 37,3 | 10,9% |
| 24 | 420 | 41,0 | 10,8% |

Dans une deuxième configuration d'incrément variable préférée, l'incrément est fixe pour une plage de pressions d'ouverture donnée mais il varie par paliers d'une plage de pressions d'ouverture à l'autre, de préférence de manière à ce que la pression d'ouverture varie avec une tendance exponentielle en fonction de la position angulaire d'indexation. Bien que moins optimisée que la configuration précédente en termes de variations de pressions d'ouverture, cette configuration par paliers permet en revanche au praticien de mémoriser facilement les incréments et les pressions d'ouverture de la valve. Par exemple, les incréments peuvent être de 10, 20 et 30 mm selon 3 plages de pressions d'ouverture croissantes (de 20 à 120 mm, de 120 à 300 mm et de 300 à 420 mm respectivement), ou les incréments peuvent être de 10, 15, 20, 25 et 30 mm selon 5 plages de pressions d'ouverture croissantes (de 20 à 100 mm, de 100 à 175 mm, de 175 à 255 mm, de 255 à 330 mm et de 330 à 420 mm respectivement). Dans ces deux exemples illustrant cette deuxième configuration, l'augmentation de la pression d'ouverture peut être, entre la position 1 (20 mm) et la position 2 (30 mm), de 50%, alors que l'augmentation de la pression d'ouverture peut être, entre la position 23 (390 mm) et la position 24 (420 mm), de 8%.

Les tableaux 3 et 4 illustrent cette configuration :

**Tableau 3**

| Position | Pression | Incrément à paliers | |
|---|---|---|---|
| | mm H₂O | mm H₂O | % variation |
| 1 | 20 | | |
| 2 | 30 | 10 | 50% |
| 3 | 40 | 10 | 33% |
| 4 | 50 | 10 | 25% |
| 5 | 60 | 10 | 20% |
| 6 | 70 | 10 | 17% |
| 7 | 80 | 10 | 14% |
| 8 | 90 | 10 | 13% |
| 9 | 100 | 10 | 11% |
| 10 | 110 | 10 | 10% |
| 11 | 120 | 10 | 9% |
| 12 | 140 | 20 | 17% |
| 13 | 160 | 20 | 14% |
| 14 | 180 | 20 | 13% |
| 15 | 200 | 20 | 11% |
| 16 | 220 | 20 | 10% |
| 17 | 240 | 20 | 9% |
| 18 | 260 | 20 | 8% |
| 19 | 280 | 20 | 8% |
| 20 | 300 | 20 | 7% |
| 21 | 330 | 30 | 10% |
| 22 | 360 | 30 | 9% |
| 23 | 390 | 30 | 8% |
| 24 | 420 | 30 | 8% |

**Tableau 4**

| Position | Pression | Incrément à paliers | |
|---|---|---|---|
| | mm H₂O | mm H₂O | % variation |
| 1 | 20 | | |
| 2 | 30 | 10 | 50% |
| 3 | 40 | 10 | 33% |
| 4 | 50 | 10 | 25% |
| 5 | 60 | 10 | 20% |
| 6 | 70 | 10 | 17% |
| 7 | 80 | 10 | 14% |
| 8 | 90 | 10 | 13% |
| 9 | 100 | 10 | 11% |
| 10 | 115 | 15 | 15% |
| 11 | 130 | 15 | 13% |
| 12 | 145 | 15 | 12% |
| 13 | 160 | 15 | 10% |
| 14 | 175 | 15 | 9% |
| 15 | 195 | 20 | 11% |
| 16 | 215 | 20 | 10% |
| 17 | 235 | 20 | 9% |
| 18 | 255 | 20 | 9% |
| 19 | 280 | 25 | 10% |
| 20 | 305 | 25 | 9% |
| 21 | 330 | 25 | 8% |
| 22 | 360 | 30 | 9% |
| 23 | 390 | 30 | 8% |
| 24 | 420 | 30 | 8% |

De préférence, le rotor peut être indexé et/ou verrouillé dans des positions angulaires prédéterminées, ces positions étant réparties sur un secteur angulaire pouvant aller jusqu'à 360°.

Le nombre de positions angulaires d'indexation est de préférence supérieur ou égal à 5, supérieur à 10, voire supérieur à 20 et/ou inférieur ou égal à 50, de préférence inférieur à 40, inférieur à 30, 24 étant considéré un nombre de positions optimal.

De préférence, les moyens de verrouillage 118 comportent 24 encoches 117 régulièrement réparties sur 360° tout autour de l'axe X, ce qui donne un angle de 15° entre chaque encoche, de sorte que le rotor peut être indexé et/ou verrouillé dans 24 positions angulaires prédéterminées, de manière à couvrir un secteur angulaire de 360°.

De préférence, les 24 positions sont disposées comme sur le cadran d'une montre « 24 heures », avec augmentation des pressions d'ouverture dans le sens horaire et diminution dans le sens anti-horaire (lorsque la valve est observée par sa face externe), de manière à ce que la position 1, correspondant à la pression d'ouverture la plus basse, soit située à 1 heure, et que la position 24, correspondant à la pression d'ouverture la plus haute, soit située à 24 heures (12 heures pour une montre normale).

Les valves représentées sur les figures comportent un tel cadran, les figures 8 à 11 correspondant aux positions 1, 6, 12 et 24, respectivement. De préférence, la valve comporte un cadran gradué radio-opaque 122 fixé sur le corps, en particulier une roue graduée, de préférence en métal, de préférence à 24 divisions, permettant, en coopération avec un indicateur radio-opaque 124 du rotor, une lecture radiographique aisée de chaque position angulaire d'indexation, et donc de la pression d'ouverture correspondante. Le cadran gradué peut être en particulier en un matériau à base de tantale ou de titane.

De préférence, les graduations du cadran et l'indicateur sont constitués par des traits ou des plots ou des bâtonnets s'étendant de préférence sensiblement radialement. Pour une lecture plus aisée, les graduations sont épaissies de préférence toute les 6, de préférence toutes les 2 divisions.

De préférence, le cadran gradué est une pièce sensiblement plate de préférence fixée, en regard du rotor 8, sur la paroi externe 126b du corps 2 qui définit la face externe 7b, de préférence sans faire saillie. En particulier, le corps peut être en un matériau synthétique, par exemple en plastique, et le cadran gradué, par exemple sous la forme d'une roue graduée, peut être logé dans une découpe ménagée sur la paroi externe 126b à cet effet.

Les graduations définissant les positions d'indexation peuvent être toutes identiques. Dans un mode de réalisation, une ou plusieurs graduations peuvent être différentes des autres, par exemple pour identifier la position correspondant à la pression d'ouverture maximale.

Dans un mode de réalisation, l'indicateur du rotor peut s'étendre, selon un rayon du rotor, de préférence de manière à rester au moins partiellement visible quelle que soit la position angulaire du rotor, et en particulier lorsque cette position correspond à une superposition partielle du bras de levier avec ledit indicateur. Cette caractéristique est particulièrement avantageuse lorsque le bras de levier présente la forme d'une plaque s'étendant sensiblement perpendiculairement à l'axe X, comme représenté sur les figures.

Lorsque le rotor comporte un dipôle magnétique formé de deux micro-aimants, l'indicateur est de préférence situé du côté du pôle Nord.

Dans un mode de réalisation, les positions du rotor peuvent évoluer de manière continue (absence d'indexation). L'évolution de la pression d'ouverture peut être sensiblement proportionnelle à l'évolution de la position angulaire du rotor, c'est-à-dire que le gradient de la pression d'ouverture en fonction de la position angulaire du rotor peut être sensiblement constant. La pression d'ouverture peut ainsi évoluer linéairement au fur et à mesure de la rotation du rotor. La pression d'ouverture peut notamment évoluer pour correspondre, dans les positions correspondant à celles du tableau 1, aux pressions mentionnées dans ce tableau (incrément fixe).

Le gradient de la pression d'ouverture en fonction de la position angulaire du rotor peut également évoluer, et de préférence augmenter, de préférence de manière exponentielle, avec la pression d'ouverture. Autrement dit, pour une même amplitude de rotation du rotor, la pression d'ouverture peut évoluer d'autant plus rapidement que la pression d'ouverture initiale est élevée. La pression d'ouverture peut notamment évoluer pour correspondre, dans les positions correspondant à celles du tableau 2, aux pressions mentionnées dans ce tableau.

Le gradient peut être également constant pour une plage de pressions d'ouverture donnée et varier par paliers d'une plage de pressions d'ouverture à l'autre, de préférence de manière à ce que la pression d'ouverture varie globalement avec une tendance exponentielle. La pression d'ouverture peut notamment évoluer pour correspondre, dans les positions correspondant à celles des tableaux 3 et 4, aux pressions mentionnées dans ces tableaux.

On se reporte à présent aux figures 1 à 4.

Le rotor définit une boutonnière 53, de préférence spiralée autour de l'axe X, de préférence ménagée dans la paroi interne 55a du rotor 8, qui s'étend sensiblement perpendiculairement à l'axe X. On distingue les profils intérieur 51 et extérieur 52 de la boutonnière 53, orientés vers l'intérieur (vers l'axe X) et vers l'extérieur, respectivement.

La boutonnière 53 peut avantageusement s'étendre sur plus de 270°, plus de 300°, plus de 300°, plus de 350°, voire sur plus de 360°, ou plus de 370°, plus de 720° ou plus de 1080° autour de l'axe X de rotation du rotor 8. De préférence, le chemin de came s'étend ainsi sur plus de 270°, plus de 300°, plus de 320°, plus de 350°, voire sur plus de 360°, ou plus de 370° ou plus de 400°, plus de 720°, voire plus de 1080° autour de l'axe du rotor. La précision de réglage en est améliorée et/ou la largeur de la plage de réglage en est augmentée.

La plage de réglage de la pression d'ouverture de la valve peut ainsi notamment s'étendre sur plus de 300 mmH₂O, plus de 400 mmH₂O, plus de 450 mmH₂O, voire plus de 500 mmH₂O, plus de 550 mmH₂O, ou même plus de 600 mmH₂O. Elle peut, par exemple, s'étendre de 10 mmH₂O à plus de 500 mmH₂O.

L'organe de rappel élastique 13 comporte un bras d'appui 57 élastique et un bras de levier 59 guidé, par l'intermédiaire du pion 61, par appui sur le profil intérieur défini par la surface latérale de la boutonnière (contact mobile), le guidage du bras de levier 59 agissant sur la contrainte exercée par le bras d'appui 57 sur la bille 11.

Le suiveur de came, en l'occurrence un pion 61 monté coulissant dans la boutonnière 53 de guidage, est en contact permanent avec le profil intérieur 51 de la boutonnière, qui définit ainsi le chemin de came 19.

Le pion 61 peut ainsi exercer une action sensiblement centrifuge sur la surface de la boutonnière.

Classiquement, pour les très faibles pressions d'ouverture, l'appui de l'organe de rappel élastique sur le rotor, et donc l'appui de l'organe de rappel élastique sur l'obturateur, peut être momentanément interrompu, par exemple en cas de choc. Avantageusement, une boutonnière assure un guidage empêchant tout déplacement latéral du suiveur de came, assurant ainsi qu'il ne peut se déplacer que le long de la boutonnière. Ainsi, le guidage par la boutonnière exerce une action assurant un appui permanent de l'organe de rappel élastique sur l'obturateur. Ce guidage est particulièrement utile lorsque l'organe de rappel élastique, et en particulier le bras de levier, présentent une grande longueur.

Les extrémités 65 et 67 de la boutonnière constituent des butées pour le pion 61, bloquant la rotation du rotor au-delà des positions extrêmes HP et BP correspondant aux pressions d'ouverture la plus élevée et la plus faible, respectivement. Elles évitent également tout « saut » de pression entre ces deux positions extrêmes.

Les figures 5 à 7 illustrent la possibilité d'avoir un chemin de came permettant une action centrifuge de l'organe de rappel élastique 13 en ménageant un évidement 50, éventuellement traversant, dans la paroi interne 55a ou externe 55b (qui sont orientées vers l'intérieur et vers l'extérieur du corps du patient, respectivement) du rotor 8, de préférence dans la paroi interne 55a, ou, de manière équivalente, en y prévoyant un bourrelet de matière.

Le chemin de came 19 présente un rayon décroissant sur un secteur angulaire sensiblement égal à 360°, dans le sens des aiguilles d'une montre, lorsque la valve est observée par sa face interne.

### Fonctionnement

Après implantation d'une valve 1 dans le corps du patient, l'opérateur règle la position angulaire du rotor.

A chaque position angulaire du rotor 8 correspond une contrainte exercée par le bras d'appui 57 sur la bille 11. Cette contrainte, exercée de manière élastique, correspond à la pression minimale nécessaire pour que le liquide céphalo-rachidien en amont de l'orifice d'entrée 5 puisse dégager, au moins partiellement, la bille 11 de l'orifice d'entrée 5 et ainsi s'écouler à l'intérieur de la chambre 3. Cette pression est appelée pression d'ouverture.

Le chemin de came 19 est en spirale, de manière que la position angulaire du pion 61 autour de l'axe Y dépende directement de la position angulaire du rotor 8 autour de l'axe X. La rotation du rotor 8 conduit ainsi à une rotation du bras de levier 59 autour de l'axe Y et, le bras d'appui 57 étant fixé sur le bras de levier 59, à une variation de la contrainte exercée par le bras d'appui 57 sur la bille 11. La pression d'ouverture est ainsi réglée par déplacement du pion 61 sur le chemin de came 19.

Le chemin de came et l'organe de rappel élastique sont disposés de telle manière qu'une rotation du rotor dans le sens des aiguilles d'une montre (par rapport à la face externe, i.e. qui sera orientée vers la peau du patient) a pour effet de rapprocher le bras de levier 59 de l'axe X du rotor 8 en fermant l'angle α formé par les deux bras de l'organe de rappel élastique, avec pour conséquence une augmentation de la contrainte exercée par le bras d'appui 57 sur la bille 11, une augmentation de la pression d'ouverture de la valve et une réduction du débit de liquide céphalo-rachidien.

Inversement, une rotation du rotor 8 dans le sens inverse des aiguilles d'une montre a pour effet d'éloigner le bras de levier de l'axe X du rotor en ouvrant l'angle α formé par les deux bras de l'organe de rappel élastique, avec pour conséquence une réduction de la contrainte exercée par le bras d'appui 57 sur la bille 11, et donc une diminution de la pression d'ouverture de la valve et par conséquent une augmentation du débit de liquide céphalo-rachidien.

Le comportement de la valve est ainsi similaire à celui d'un robinet qu'actionnerait l'opérateur : Il ferme le robinet en tournant le rotor dans le sens des aiguilles d'une montre, jusqu'à atteindre la butée de la position 24 correspondant à une position de quasi fermeture (position « virtual OFF » à plus de 400 mm H₂O). Il ouvre le robinet en tournant le rotor dans le sens inverse des aiguilles d'une montre, jusqu'à atteindre la butée de la position 1 correspondant à une position de quasi ouverture totale (très basse pression).

Le liquide céphalo-rachidien ayant pénétré dans la chambre 3 transite jusqu'à l'orifice de sortie 6 puis est évacué. Lorsque la quantité de liquide évacuée est suffisante, la pression de liquide céphalo-rachidien en amont de l'orifice d'ouverture 5 diminue jusqu'à devenir inférieure à la pression d'ouverture, ce qui conduit le bras d'appui 57 à repousser la bille 11 sur son siège de manière à obturer l'orifice d'entrée 5 et ainsi stopper l'évacuation du liquide céphalo-rachidien.

En cas de besoin, l'opérateur peut modifier facilement la position angulaire du rotor 8, et ainsi régler la pression d'ouverture.

Comme cela apparaît clairement à présent, une valve 1 selon la présente invention permet un réglage précis de la pression d'ouverture, sur une plage très large, en particulier lorsque le chemin de came s'étend sur plus de 360°.

Bien entendu, l'invention n'est pas limitée aux exemples de mise en oeuvre qui viennent d'être décrits.

La présente invention peut être adaptée à tout type de valve à pression ajustable. Selon un mode de réalisation avantageux, elle est adaptée à une valve ajustable à verrouillage magnétique, telle que décrite dans le brevet US 5,643,194 ou EP 0688 575, ou le brevet EP 1 604 703 ou US 7,758,536.

Le chemin de came n'est pas nécessairement traversant, comme lorsqu'il est défini par la boutonnière 53 ou l'évidement 50. En particulier, la boutonnière peut être remplacée par une rainure.

L'organe de rappel élastique peut être de tout type.

On ne sort pas du cadre de la présente invention lorsque le chemin de came présente une pluralité d'encoches réparties, par exemple régulièrement, autour de l'axe X.

Ces encoches peuvent être configurées de manière à correspondre à des positions angulaires d'indexation du rotor.

L'organe de rappel élastique peut être conformé pour pouvoir s'engager dans ces encoches.

On peut également prévoir un chemin de came qui s'étend seulement sur un secteur angulaire inférieur à 360°, et par exemple sur un secteur angulaire d'environ 180°.

Les évolutions pour la pression d'ouverture décrites dans le cadre d'une valve comportant des positions angulaires d'indexation prédéterminées peuvent être appliquées à tout type de valve, et en particulier à des valves permettant une évolution continue de la pression d'ouverture. La pente du chemin de came peut être facilement adaptée à cet effet.

Les caractéristiques des différents modes de réalisation, en particulier des modes de réalisation représentés, peuvent être combinées, sauf incompatibilité technique.

### REFERENCES DES FIGURES

1 : valve de drainage
2 : corps de valve
3 : chambre
5 : orifice d'entrée du LCR
6 : orifice de sortie du LCR
7a et 7b : faces interne et externe du corps 2
8 : rotor
9 : pivot du rotor
11 : bille (obturateur)
13 : organe de rappel élastique
14 : pivot de l'organe de rappel élastique
15 : conduit d'entrée
17 : conduit de sortie
18 : surface latérale extérieure du rotor 8
19 : chemin de came
50 : évidement du rotor
51: profil intérieur de la boutonnière 53 ou de l'évidement 50
52 : profil extérieur de la boutonnière 53
53 : boutonnière
55a et 55b : parois interne et externe du rotor, respectivement
57 : bras d'appui sur l'obturateur
59 : bras de levier
61 : pion (ou plot) en contact avec la surface de came
65 et 67 : extrémités de la boutonnière 53 ou de l'évidement 50 définissant des butées en pressions haute et basse
110 et 111 : logements des micro-aimants
112 et 113 : micro-aimants
115 : relief de verrouillage (ergot)
117 : encoches
118 : moyens de verrouillage
119 : partie centrale fixe supportant les encoches
122 : cadran gradué radio-opaque
124 : indicateur radio-opaque du rotor
126b : paroi externe du corps 2
X : axe de rotation du rotor
Y : axe de pivotement de l'organe de rappel élastique
α : angle formé par les deux bras de l'organe de rappel élastique

## Revendications

1. Valve de drainage (1) destinée à être implantée sous la peau d'un patient et à drainer du liquide céphalo-rachidien, ladite valve comportant :
- un corps (2) définissant une chambre (3) dans laquelle débouchent un orifice d'entrée (5) et un orifice de sortie (6) du liquide céphalo-rachidien,
- un obturateur (11) apte à obturer au moins partiellement, voire totalement, l'orifice d'entrée (5),
- un organe de rappel élastique (13) agencé pour exercer une contrainte sur l'obturateur (11), de manière à le pousser, de manière élastique, vers l'orifice d'entrée de manière que le passage de liquide céphalo-rachidien par l'orifice d'entrée ne soit possible que si sa pression est supérieure ou égale à une pression d'ouverture,
- un rotor (8) logé dans la chambre, apte à tourner autour d'un axe X entre deux positions extrêmes et comportant un chemin de came (19) sur laquelle prend appui l'organe de rappel élastique (13) de sorte que la contrainte exercée par l'organe de rappel élastique (13) sur l'obturateur (11) est modifiée par la rotation du rotor (8),
ladite valve étant **caractérisée en ce que** le chemin de came est défini par un profil intérieur du rotor, orienté vers l'axe X, de manière que l'organe de rappel élastique (13) exerce, sur le chemin de came (19) du rotor (8), une force dite «force de traction », comportant une composante centrifuge par rapport à l'axe X.

2. Valve selon la revendication précédente, le chemin de came (19) s'étendant sur plus de 320° autour de l'axe X de rotation du rotor (8).

3. Valve selon la revendication précédente, le chemin de came (19) s'étendant sur plus de 370° autour de l'axe X de rotation du rotor (8).

4. Valve selon l'une quelconque des revendications précédentes, le chemin de came étant adapté de manière à assurer des première et deuxième butées de l'organe de rappel élastique empêchant la poursuite de la rotation du rotor au-delà de première et deuxième positions extrêmes, dans les premier et deuxième sens de rotation du rotor, respectivement.

5. Valve selon l'une quelconque des revendications précédentes, dans laquelle le chemin de came ne présente pas de rupture de pente, de préférence s'étend en spirale autour de l'axe X, la pente étant de préférence sensiblement constante.

6. Valve selon l'une quelconque des revendications précédentes, dans laquelle l'organe de rappel élastique est monté à rotation, par rapport au corps (2), autour d'un axe Y sensiblement parallèle à l'axe X et comporte un bras de levier en contact mobile avec le chemin de came et un bras d'appui sur l'obturateur, les bras du levier et d'appui étant reliés entre eux par un pivot passant par l'axe Y,
le bras de levier (59) formant avec le bras d'appui (57) un secteur d'angle (a) coupant l'axe X,
la direction du bras de levier étant définie, lorsque la valve est observée suivant l'axe X, par la droite passant par l'axe Y et par le point d'appui du bras de levier sur le chemin de came, et
la direction du bras d'appui étant définie, lorsque la valve est observée suivant l'axe X, par la droite passant par l'axe Y et le point d'appui du bras d'appui sur l'obturateur.

7. Valve selon la revendication immédiatement précédente, dans laquelle le bras de levier en contact mobile avec le chemin de came comporte une tige rigide ou flexible, mobile et/ou déformable dans un plan perpendiculaire à l'axe X, de préférence distinct et de préférence parallèle à un plan perpendiculaire de l'axe X et passant par le chemin de came, et un suiveur de came, sensiblement axial, en contact mobile avec le chemin de came.

8. Valve selon l'une quelconque des revendications précédentes, dans laquelle ledit organe de rappel élastique exerce ladite force de traction pour l'ensemble des positions angulaires dudit rotor.

9. Valve selon l'une quelconque des revendications précédentes, le chemin de came étant défini par une cavité ménagée dans le rotor, en particulier définie par une boutonnière (53), une rainure ou un évidement (50), ou par un bourrelet de matière.

10. Valve selon l'une quelconque des revendications précédentes, l'organe de rappel élastique et le rotor étant conformés pour autoriser le réglage d'une pression d'ouverture de la valve sur une plage d'une amplitude supérieure à 200 mmH₂O, voire supérieure à 350 mmH₂O.

11. Valve selon l'une quelconque des revendications précédentes, le rotor comportant un dipôle magnétique formé de deux micro-aimants fixes ou mobiles linéairement par rapport au rotor suivant une direction sensiblement radiale par rapport à l'axe X et aptes à coopérer avec des moyens de verrouillage du rotor de manière à verrouiller ledit rotor dans une pluralité de positions angulaires d'indexation prédéterminées.

12. Valve selon l'une quelconque des revendications précédentes, dans laquelle, sur au moins une plage de pressions d'ouverture,
- le rotor peut être positionné dans une pluralité de positions angulaires d'indexation prédéterminées et l'incrément entre deux pressions d'ouverture correspondant à deux positions angulaires du rotor successives est constant, ou
- les positions du rotor peuvent évoluer de manière continue et le gradient de la pression d'ouverture en fonction de la position angulaire du rotor est constant.

13. Valve selon l'une quelconque des revendications 1 à 11, dans laquelle, sur au moins une plage de pressions d'ouverture,
- le rotor peut être positionné dans une pluralité de positions angulaires d'indexation prédéterminées et l'incrément entre deux pressions d'ouverture correspondant à deux positions angulaires du rotor successives est variable, ou
- les positions du rotor peuvent évoluer de manière continue et le gradient de la pression d'ouverture en fonction de la position angulaire du rotor est variable.

14. Valve selon la revendication précédente, dans laquelle ledit incrément ou ledit gradient évolue de manière sensiblement exponentielle au fur et à mesure de la rotation du rotor.

15. Valve selon l'une quelconque des revendications précédentes, dans laquelle le chemin de came est conformé pour définir plusieurs plages de pressions d'ouverture, la pression d'ouverture évoluant de manière différente en fonction de la plage de pressions d'ouverture considérée.

16. Valve selon la revendication immédiatement précédente, dans laquelle
- le rotor peut être positionné dans une pluralité de positions angulaires d'indexation prédéterminées, l'incrément entre deux pressions d'ouverture correspondant à deux positions angulaires d'indexation successives étant constant dans plusieurs plages de positions angulaires du rotor successives, l'incrément augmentant, de préférence de manière exponentielle, d'une plage à la suivante, ou
- les positions du rotor peuvent évoluer de manière continue et le gradient de la pression d'ouverture en fonction de la position angulaire du rotor est constant dans plusieurs plages de positions angulaires du rotor successives, ledit gradient augmentant, de préférence de manière exponentielle, d'une plage à la suivante.

17. Valve selon l'une quelconque des revendications précédentes, dans laquelle l'organe de rappel élastique comporte soit un bras de levier rigide et un bras d'appui flexible, de préférence sous la forme d'un ressort à lame, de préférence courbe, soit un bras de levier flexible, de préférence sous la forme d'une tige flexible, et un bras d'appui rigide, de préférence sous la forme d'une lame rigide, de préférence courbe.

18. Valve selon l'une quelconque des revendications précédentes, dans laquelle le rotor peut être verrouillé dans vingt-quatre positions angulaires prédéterminées, séparées entre elles de 15°, une rotation dans le sens horaire du rotor, lorsque la face externe du rotor est observée, conduisant à une augmentation de la pression d'ouverture.

19. Valve selon l'une quelconque des revendications précédentes, dans laquelle l'axe de rotation du rotor est excentré par rapport au centre de la chambre dans laquelle le rotor est logé et/ou par rapport à l'axe reliant les orifices d'entrée et de sortie du liquide céphalo-rachidien.

## Patentansprüche

1. Drainageventil (1), das dazu bestimmt ist, unter der Haut eines Patienten implantiert zu werden und Gehirn- und Rückenmarksflüssigkeit abzuleiten, wobei das Ventil aufweist:
- ein Gehäuse (2), das eine Kammer (3) definiert, in welche eine Eintrittsöffnung (5) und eine Austrittsöffnung (6) für die Gehirn- und Rückenmarksflüssigkeit münden,
- einen Verschluss (11), der geeignet ist, die Eintrittsöffnung (5) wenigstens teilweise oder sogar vollständig zu verschließen,
- ein elastisches Rückholorgan (13), das dafür ausgelegt ist, auf den Verschluss (11) eine Spannung auszuüben, um ihn auf elastische Weise in Richtung der Eintrittsöffnung zu schieben, derart, dass der Durchfluss von Gehirn- und Rückenmarksflüssigkeit durch die Eintrittsöffnung nur möglich ist, wenn der Druck größer als ein oder gleich einem Öffnungsdruck ist,
- einen in der Kammer aufgenommenen Rotor (8), der geeignet ist, sich um eine Achse X zwischen zwei Endpositionen zu drehen, und eine Nockenbahn (19) aufweist, auf welcher sich das elastische Rückholorgan (13) abstützt, derart, dass die Spannung, die von dem elastischen Rückholorgan (13) auf den Verschluss (11) ausgeübt wird, durch die Drehung des Rotors (8) geändert wird,
wobei das Ventil **dadurch gekennzeichnet ist, dass** die Nockenbahn durch ein inneres Profil des Rotors definiert ist, das der Achse X zugewandt ist, derart, dass das elastische Rückholorgan (13) auf die Nockenbahn (19) des Rotors (8) eine "Zugkraft" genannte Kraft ausübt, die eine bezüglich der Achse X zentrifugale Komponente aufweist.

2. Ventil nach dem vorhergehenden Anspruch, wobei sich die Nockenbahn (19) über mehr als 320° um die Drehachse X des Rotors (8) herum erstreckt.

3. Ventil nach dem vorhergehenden Anspruch, wobei sich die Nockenbahn (19) über mehr als 370° um die Drehachse X des Rotors (8) herum erstreckt.

4. Ventil nach einem der vorhergehenden Ansprüche, wobei die Nockenbahn so beschaffen ist, dass ein erster und ein zweiter Anschlag des elastischen Rückholorgans sichergestellt sind, welche die Fortsetzung der Drehung des Rotors über die erste und die zweite Endposition hinaus in der ersten bzw. zweiten Drehrichtung des Rotors verhindern.

5. Ventil nach einem der vorhergehenden Ansprüche, wobei die Nockenbahn keine plötzliche Änderung der Neigung aufweist und sich vorzugsweise spiralförmig um die Achse X erstreckt, wobei die Neigung vorzugsweise im Wesentlichen konstant ist.

6. Ventil nach einem der vorhergehenden Ansprüche, wobei das elastische Rückholorgan bezüglich des Gehäuses (2) drehbar um eine Achse Y gelagert ist, die im Wesentlichen parallel zur Achse X ist, und einen Hebelarm, der sich in beweglichem Kontakt mit der Nockenbahn befindet, und einen Arm zur Abstützung auf dem Verschluss aufweist, wobei der Hebelarm und der Stützarm durch einen Drehzapfen miteinander verbunden sind, der durch die Achse Y verläuft,
wobei der Hebelarm (59) mit dem Stützarm (57) einen Sektor mit einem Winkel (α) bildet, der die Achse X schneidet,
wobei die Richtung des Hebelarmes, wenn das Ventil entlang der Achse X betrachtet wird, durch die Gerade definiert ist, die durch die Achse Y und durch den Punkt der Abstützung des Hebelarmes auf der Nockenbahn verläuft, und
wobei die Richtung des Stützarmes, wenn das Ventil entlang der Achse X betrachtet wird, durch die Gerade definiert ist, die durch die Achse Y und den Punkt der Abstützung des Stützarmes auf dem Verschluss verläuft.

7. Ventil nach dem unmittelbar vorhergehenden Anspruch, wobei der Hebelarm, der sich in beweglichem Kontakt mit der Nockenbahn befindet, eine starre oder flexible Stange, die beweglich und/oder verformbar in einer zur Achse X senkrechten Ebene ist, welche vorzugsweise verschieden von und vorzugsweise parallel zu einer zur Achse X senkrechten und durch die Nockenbahn verlaufenden Ebene ist, und einen im Wesentlichen axialen Nockenfolger, der sich in beweglichem Kontakt mit der Nockenbahn befindet, umfasst.

8. Ventil nach einem der vorhergehenden Ansprüche, wobei das elastische Rückholorgan die Zugkraft für sämtliche Winkelpositionen des Rotors ausübt.

9. Ventil nach einem der vorhergehenden Ansprüche, wobei die Nockenbahn durch einen Hohlraum definiert ist, der in dem Rotor ausgebildet ist, insbesondere durch ein Langloch (53), eine Nut oder eine Ausnehmung (50), oder durch einen Materialwulst.

10. Ventil nach einem der vorhergehenden Ansprüche, wobei das elastische Rückholorgan und der Rotor dafür ausgebildet sind, die Regelung eines Öffnungsdrucks des Ventils in einem Bereich mit einer Breite zu ermöglichen, die größer als 200 mm H₂O oder sogar größer als 350 mm H₂O ist.

11. Ventil nach einem der vorhergehenden Ansprüche, wobei der Rotor einen magnetischen Dipol aufweist, der von zwei Mikromagneten gebildet wird, die bezüglich des Rotors fest oder linear beweglich entlang einer bezüglich der Achse X im Wesentlichen radialen Richtung sind und geeignet sind, mit Verriegelungsmitteln des Rotors so zusammenzuwirken, dass der Rotor in mehreren vorbestimmten Einrastwinkelpositionen verriegelt wird.

12. Ventil nach einem der vorhergehenden Ansprüche, wobei in wenigstens einem Bereich von Öffnungsdrücken
- der Rotor in mehreren vorbestimmten Einrastwinkelpositionen positioniert werden kann und das Inkrement zwischen zwei Öffnungsdrücken, die zwei aufeinander folgenden Winkelpositionen des Rotors entsprechen, konstant ist, oder
- die Positionen des Rotors sich stetig ändern können und der Gradient des Öffnungsdruckes als Funktion der Winkelposition des Rotors konstant ist.

13. Ventil nach einem der Ansprüche 1 bis 11, wobei in wenigstens einem Bereich von Öffnungsdrücken
- der Rotor in mehreren vorbestimmten Einrastwinkelpositionen positioniert werden kann und das Inkrement zwischen zwei Öffnungsdrücken, die zwei aufeinander folgenden Winkelpositionen des Rotors entsprechen, variabel ist, oder
- die Positionen des Rotors sich stetig ändern können und der Gradient des Öffnungsdruckes als Funktion der Winkelposition des Rotors variabel ist.

14. Ventil nach dem vorhergehenden Anspruch, wobei sich das Inkrement oder der Gradient während der Rotation des Rotors exponentiell ändert.

15. Ventil nach einem der vorhergehenden Ansprüche, wobei die Nockenbahn so ausgebildet ist, dass mehrere Bereiche von Öffnungsdrücken definiert werden, wobei sich der Öffnungsdruck in Abhängigkeit von dem betrachteten Bereich von Öffnungsdrücken auf unterschiedliche Weise ändert.

16. Ventil nach dem unmittelbar vorhergehenden Anspruch, wobei
- der Rotor in mehreren vorbestimmten Einrastwinkelpositionen positioniert werden kann, wobei das Inkrement zwischen zwei Öffnungsdrücken, die zwei aufeinander folgenden Einrastwinkelpositionen entsprechen, in mehreren Bereichen von aufeinander folgenden Winkelpositionen des Rotors konstant ist, wobei sich das Inkrement von einem Bereich zum folgenden vergrößert, vorzugsweise exponentiell, oder
- die Positionen des Rotors sich stetig ändern können und der Gradient des Öffnungsdruckes als Funktion der Winkelposition des Rotors in mehreren Bereichen von aufeinander folgenden Winkelpositionen des Rotors konstant ist, wobei sich der Gradient von einem Bereich zum folgenden erhöht, vorzugsweise exponentiell.

17. Ventil nach einem der vorhergehenden Ansprüche, wobei das elastische Rückholorgan entweder einen starren Hebelarm und einen flexiblen Stützarm, vorzugsweise in Form einer Blattfeder, die vorzugsweise gekrümmt ist, oder einen flexiblen Hebelarm, vorzugsweise in Form einer flexiblen Stange, und einen starren Stützarm, vorzugsweise in Form eines starren Blattes, das vorzugsweise gekrümmt ist, aufweist.

18. Ventil nach einem der vorhergehenden Ansprüche, wobei der Rotor in vierundzwanzig vorbestimmten Winkelpositionen verriegelt werden kann, die jeweils um 15° voneinander beabstandet sind, wobei eine Drehung des Rotors im Uhrzeigersinn, von der Außenseite des Rotors aus gesehen, zu einer Erhöhung des Öffnungsdrucks führt.

19. Ventil nach einem der vorhergehenden Ansprüche, wobei die Drehachse des Rotors exzentrisch bezüglich des Mittelpunktes der Kammer, in welcher der Rotor aufgenommen ist, und/oder bezüglich der die Eintrittsöffnung und die Austrittsöffnung für die Gehirn- und Rückenmarksflüssigkeit verbindenden Achse ist.

## Claims

1. A drainage valve (1) intended to be implanted under the skin of a patient and to drain cerebrospinal fluid, said valve comprising:
- a body (2) defining a chamber (3) into which there open an inlet orifice (5) and an outlet orifice (6) for the cerebrospinal fluid,
- a shutter (11) able to shut off the inlet orifice (5) at least partially, or even fully,
- an elastic return member (13) designed to exert an influence on the shutter (11) so as to push it, elastically, toward the inlet orifice so that the passage of cerebrospinal fluid through the inlet orifice is possible only if its pressure is greater than or equal to an opening pressure,
- a rotor (8) housed in the chamber, able to rotate about an axis X between two extreme positions and comprising a camway (19) against which the elastic return member (13) bears so that the influence exerted by the elastic return member (13) on the shutter (11) is modified by the rotation of the rotor (8),
the valve being **characterized in that** the camway is defined by an interior profile of the rotor, directed toward the axis X, so that the elastic return member (13) exerts, on the camway (19) of the rotor (8), a force referred to as a "pulling force" comprising a component that is centrifugal with respect to the axis X.

2. The valve as claimed in the preceding claim, the camway (19) extending over more than 320° about the axis X of rotation of the rotor (8).

3. The valve as claimed in the preceding claim, the camway (19) extending over more than 370° about the axis X of rotation of the rotor (8).

4. The valve as claimed in any one of the preceding claims, the camway being designed to provide first and second end stops for the elastic return member preventing the rotor from rotating beyond first and second extreme positions, in the first and second directions of rotation of the rotor, respectively.

5. The valve as claimed in any one of the preceding claims, in which the camway has no break in slope, preferably extends in a spiral about the axis X, the gradient preferably being substantially constant.

6. The valve as claimed in any one of the preceding claims, in which the elastic return member is mounted to rotate, with respect to the body (2), about an axis Y substantially parallel to the axis X and comprises a lever arm in mobile contact with the camway and a bearing arm pressing against the shutter, the lever and bearing arms being connected to one another by a pivot passing through the axis Y,
the lever arm (59) forming, with the bearing arm (57), a sector of angle (α) intersecting the axis X,
the direction of the lever arm being defined, when the valve is observed along the axis X, by the straight line passing through the axis Y and by the point at which the lever arm bears against the camway, and
the direction of the bearing arm being defined, when the valve is observed along the axis X, by the straight line passing through the axis Y and the point at which the bearing arm presses against the shutter.

7. The valve as claimed in the immediately preceding claim, in which the lever arm in mobile contact with the camway comprises a rigid or flexible rod, which is able to move and/or deform in a plane perpendicular to the axis X, preferably distinct from and preferably parallel to a plane perpendicular to the axis X and passing through the camway, and a substantially axial cam follower in mobile contact with the camway.

8. The valve as claimed in any one of the preceding claims, in which said elastic return member exerts said pulling force for all angular positions of said rotor.

9. The valve as claimed in any one of the preceding claims, the camway being defined by a cavity formed in the rotor, particularly defined by a slot (53), a groove or a recess (50), or by a raised run of material.

10. The valve as claimed in any one of the preceding claims, the elastic return member and the rotor being configured to allow a valve opening pressure to be set in a range of an amplitude greater than 200 mmH₂O, or even greater than 350 mmH₂O.

11. The valve as claimed in any one of the preceding claims, the rotor comprising a magnetic dipole formed of two micromagnets that are fixed or linearly mobile with respect to the rotor in a direction substantially radial with respect to the axis X and able to collaborate with rotor locking means so as to lock said rotor in a plurality of predetermined angular indexing positions.

12. The valve as claimed in any one of the preceding claims, in which, in at least one range of opening pressures,
- the rotor may be positioned in a plurality of predetermined angular indexing positions and the increment between two opening pressures corresponding to two successive angular positions of the rotor is constant, or
- the positions of the rotor may evolve continuously and the gradient of opening pressure as a function of the angular position of the rotor is constant.

13. The valve as claimed in any one of claims 1 to 11, in which, in at least one range of opening pressures,
- the rotor may be positioned in a plurality of predetermined angular indexing positions and the increment between two opening pressures corresponding to two successive angular positions of the rotor is variable, or
- the positions of the rotor may evolve continuously and the gradient of the opening pressure as a function of the angular position of the rotor is variable.

14. The valve as claimed in the preceding claim, in which said increment or said gradient evolves substantially exponentially as the rotor rotates.

15. The valve as claimed in any one of the preceding claims, in which the camway is configured to define several ranges of opening pressures, the opening pressure evolving differently according to which range of opening pressures is considered.

16. The valve as claimed in the immediately preceding claim, in which
- the rotor may be positioned in a plurality of predetermined angular indexing positions, the increment between two opening pressures corresponding to two successive angular indexing positions being constant in several ranges of successive angular positions of the rotor, the increment increasing, preferably exponentially, from one range to the next, and
- the positions of the rotor may evolve continuously and the gradient of opening pressure as a function of the angular position of the rotor is constant in several ranges of successive angular positions of the rotor, said gradient increasing, preferably exponentially, from one range to the next.

17. The valve as claimed in any one of the preceding claims, in which the elastic return member comprises either a rigid lever arm and a flexible bearing arm, preferably in the form of a leaf spring, preferably curved, or a flexible lever arm, preferably in the form of a flexible rod, and a rigid bearing arm, preferably in the form of a rigid leaf, preferably curved.

18. The valve as claimed in any one of the preceding claims, in which the rotor may be locked in twenty-four predetermined angular positions separated from one another by 15°, a rotation of the rotor in the clockwise direction, when looking at the external face of the rotor, leading to an increase in the opening pressure.

19. The valve as claimed in any one of the preceding claims, in which the axis of rotation of the rotor is off-centered with respect to the center of the chamber in which the rotor is housed and/or with respect to the axis connecting the cerebrospinal fluid inlet and outlet orifices.
